# EUROPEAN PATENT APPLICATION

(11) **EP 1 955 695 A1**
(43) Date of publication of application: **13.08.2008**
(21) Application number: 07002521.8
(22) Date of filing: 06.02.2007
(51) Int. Cl.: A61K 9/51

(54) **Nanocapsules of lipophilic complexes of nucleic acids**

(71) Applicant: INSTITUT NATIONAL DE LA SANTE ET DE LA RECHERCHE MEDICALE (INSERM), 75654 Paris Cédex 13 (FR)
(72) Inventor: The designation of the inventor has not yet been filed
(74) Representative: Le Coupanec, Pascale A.M.P.

(57) **Abstract**

The present invention is related to a nanocapsule comprising at least:
- an external lipid layer, solid at room temperature,
- an oily core, liquid room temperature,
said oily core containing at least one lipophilic complex of at least one nucleic acid sequence, said complex having a positive/negative charges ratio of at least 0.5

## Description

The instant invention relates to new nanocapsules comprising a nucleic acid sequence, a process for obtaining such nanocapsules and the use of such nanocapsules for the manufacture of pharmaceutical compositions for gene or antisense therapy or gene silencing.

Gene therapy or antisense therapy have for purpose the insertion of a nucleic acid sequence into specific cells or tissues for treating or relieving a disease condition. The inserted nucleic acid sequence may either have for purpose to compensate a defecting gene or to reduce or suppress the overexpression of a gene.

Accordingly, efficient and accurate systemic delivery of nucleic acids sequences to a specific tissue or organ is of crucial importance for the future development of a nucleic acid-based therapy in various pathologies where organ or tissue injection cannot be envisaged.

Numerous methods are known to introduce a nucleic acid sequence into a host cell. Those methods may be mainly divided into two categories the definitions of which relying upon the use of viral or non viral (or synthetic) vectors.

Synthetic vectors represent a good alternative to viral vectors due to their easiness of production, their absence of risk associated with replication-competent virus and of the size limitation of the transgene. They have proven their efficacy in promoting nucleic acid transfer in cultured cells.

As examples of commonly used synthetic vectors devised for nucleic acids transfection, lipoplexes may be cited which are complexes of nucleic acids and cationic lipids, with optionally neutral lipids.

Lipoplexes may additionally be encapsulated as described in US 6,632,671. But typically such processes require numerous and complexes steps questioning the efficiency of the method.

Unfortunately, compared to viral vectors, synthetic vectors remain less efficient *in vivo* to target a tissue after intravenous injection. Furthermore, their *in vivo* use still remains a problem because they become unstable and toxic in a biological environment.

At last, after administration of lipoplexes, the excess of cationic charge necessary to complex DNA molecules may create interaction with blood proteins such as albumin which destabilize them, as well as with complement proteins or with cells of the mononuclear phagocyte system which participate to their destabilization and their blood removal (Plank et al., Hum. Gene Ther., 1996, 7(12):1437).

Therefore, there is still a need in systemic gene or antisense therapy for synthetic vectors which may overcome the above-mentioned inconveniences.

Furthermore, there is still a need for an efficient nucleic acid sequences delivery system that may fulfil the high constraints of the blood environment.

There is also a need of a nucleic acid sequences delivery system displaying low toxicity, *in vivo* stability, as well as stealth and efficient targeting properties.

There is also a need of a nucleic acid sequences delivery system which may be obtained with high efficiency through a simple process.

The instant invention has for purpose to comply with these requirements.

Accordingly, a first object of the present invention is a nanocapsule comprising at least:
- an external lipid layer, solid at room temperature,
- an oily core liquid at room temperature,
said oily core containing at least one lipophilic complex of at least one nucleic acid sequence, said complex having a positive/negative charges ratio of at least 0.5.

Within the meaning of the invention, the term "nanocapsules" is to be understood as meaning particles comprising a core that is liquid at room temperature, coated with a film that is solid at room temperature, as opposed to nanospheres, which are matrix particles, i.e. particles whose entire mass is solid.

Within the meaning of the invention, "room temperature" is to be understood as being a temperature ranging from about 15 to about 25°C, and in particular being of about 20°C.

Within the meaning of the invention, the term "about" is understood to mean the approximation associated with a value obtained by experimental measure, as usually acknowledged in a given technical field.

Surprisingly, the inventors have observed that it was possible to introduce nucleic acid sequences complexed with cationic lipids and neutral lipids into nanocapsules comprising an external lipid layer solid at room temperature, and an oily core liquid at room temperature and to obtain nanocapsules displaying size, stability and charge surface properties convenient for gene or antisense or gene silencing therapy.

The obtained nanocapsules may have a size of about 110 ± 55 nm in diameter, a zeta potential near 5 ± 0.4 mV and an isoelectric point p*I* near 7.50 ± 0.13 which confers to them particular advantages for systemic administration.

Advantageously, a nucleic acid sequence, in particular a DNA sequence, entrapped inside said nanocapsules is efficiently protected against environmental factors of degradations, and in particular against nuclease degradation.

When injected into a mammal, the nanocapsules of the invention display low complement system activation, increased blood half-life and high take-up by tissues, allowing for a high yielding of gene expression, for example.

Furthermore, the external layer of nanoparticles of the invention may be advantageously configured to be derivatized with targeting ligands allowing to increase the accuracy and efficiency of organ or tissue targeting

According to a second aspect, the instant invention relates to a use of a nanocapsule comprising (a) an external layer solid at room temperature and (b) an oily core liquid at room temperature, for encapsulating at least one lipophilic complex of at least one nucleic acid sequence, said complex having a positive/negative charges ratio of at least 0.5.

According to another aspect, the instant invention is directed to a method for introducing at least one nucleic acid sequence into a host cell comprising at least a step of : contacting a nanocapsule of the invention with a host cell in conditions suitable for the entry of a nucleic acid sequence comprised into said nanocapsule into said host cell.

A method of the invention may be carried out *in vitro, ex vivo, in situ* and/or *in vivo.*

According to another aspect, the instant invention is directed to a pharmaceutical composition comprising in a pharmaceutically acceptable medium at least one nanocapsule of the invention.

According to another aspect, the instant invention is directed to a use of a nanocapsule of the invention for the manufacture of a pharmaceutical composition, in particular a pharmaceutical composition intended to be used in gene or antisense or gene silencing therapy.

According to another aspect, the instant invention is directed to a process for obtaining a nanocapsule of the invention comprising at least the steps of:
a) preparing an oil/water emulsion comprising at least an oily fatty phase, a lipophilic surfactant solid at 20°C, a non-ionic hydrophilic surfactant and an aqueous dispersion of lipophilic complexes of at least one nucleic acid sequence, said complex having a positive/negative charge ratio of at least 0.5,
b) subjecting the emulsion of step a) to a cycle of temperature comprising an increase of temperature up to a temperature T₂ greater than a phase inversion temperature for obtaining a water/oil emulsion, followed with a decrease of temperature down to T₁, lower than the phase inversion temperature, said cycle of temperature inducing a phase inversion of said emulsion,
c) reiterating at least one cycle of temperature according to step b),
d) subjecting the emulsion obtained at step c) to a fast cooling-dilution step with water at 0 °C for obtaining a nanocapsule in water.

According to another aspect, the instant invention is directed to a method of treatment of an individual with a condition in need thereof and/or of reducing the risk of occurrence of said condition, comprising the administration of a therapeutically effective amount of nanocapsules of the invention comprising a nucleic acid sequence suitable for treating and/or reducing the risk of occurrence of said condition.

Within the meaning of the invention, the term "therapeutically effective amount" is intended to mean the minimum amount required to observe an effect with respect to a treatment and/or a reducing of a risk of occurrence of a disease condition.

### Complex of nucleic acid

According to an embodiment, a non-encapsulated lipophilic complex in accordance with the invention may present a zeta potential ranging from 40 to 60 mV, in particular ranging from 45 to 55 mV, and more particularly ranging from 48 to 52 mV.

A zeta potential may be measured according to a protocol as described below.

According to an embodiment, a complex of the invention may have a positive/negative charges ratio of at least 0.5, and in particular ranging from 0.5:1 to 10:1, in particular from 1:1 to 5:1, and more particularly being of 5:1.

According to an embodiment, the ratio of positive to negative charge in a complex suitable for the invention is such that it may have a global charge near the neutrality.

According to an embodiment, a non-encapsulated lipophilic complex may have an average size ranging from 400 to I µm, in particular ranging from 600 to 800 nm, and more particularly being of about 700 nm.

However, it has to be noticed that prior encapsulation, lipophilic complex are generally under aggregate form,

After being encapsulated to provide a nanocapsule according to the invention, the size of the above-defined complex may be reduced by a factor of about 7 to 20 and may range from 30 to 150 nm in diameter, in particular ranging from 40 to 120 nm, and more particularly ranging from 50 to 100 nm in diameter.

According to an embodiment, a lipophilic complex may be present in a nanocapsule of the invention in an amount of up to 50%, in particular ranging from 7 to 35%, and more particularly ranging from 10 to 25% by weight relative to the total weight of the nanocapsule.

According to an embodiment, a lipophilic complex of at least one nucleic acid sequence in accordance with the invention may be a lipoplex.

As defined in the art, a lipoplex is a liposome comprising a nucleic acid sequence.

The liposome may comprise at least one cationic lipid and optionally at least one neutral lipid.

A cationic lipid useful for the invention may comprise at least one ammonium moiety.

The hydrocarbon moieties of a cationic lipid useful for the invention may be derived from alkyl chain, fatty acid, fatty alcool or fatty amine, in C₈-C₂₀, in particular in C₁₂-C₁₈, and more particularly in C₁₆.

According to an embodiment, a cationic lipid suitable for the invention may be chosen from 1,2-dioleyloxy-3-trimethyl ammonium chloride propane (DOTAP), (N-(1-(2,3-dioleyloxy)propyl)-N,N,N-triméthylammonium chloride (DOTMA), N-[1]-(2,3-dimyristyloxy)propyl]N,N-dimethyl-N-(2hydroxyethyl)ammonium chloride (DMRLE), 2,3-dioleyloxy-N-2-(carboxamidospermine)ethyl-N,N-dimethyl-1-propammonium chloride (DOSPA), dioctadecyl amidoglycinespermine (DOGS), DODAP (1,2-dioleoyl-3-(dimethylamino)propane), DODAB (dioctadecyldimethylammonium bromide), cetyl trimethylammonium bromide (CTAB), 1,2-dipalmitoylephosphatidyleethanolamidospermine (1-DPPES), aminoglycoside lipidic derivatives and mixtures thereof.

According to an embodiment, a composition of lipoplexes of the invention may comprise a cationic lipid in an amount ranging from 0.5 mg to 14 mg, in particular from 1 to 10 mg, and more particularly from 4 to 8 mg.

According to an embodiment, a composition of lipoplexes of the invention may comprise from 0.05 to 1%, and in particular from 0.1 to 0.5 % of cationic lipid by weight relative to the total weight of the composition.

Neutral lipids that may convene for the invention may be chosen from L-a-dioleylphosphatidylethanolamine (DOPE), dioleylphosphatidylcholine (DOPC), cholesterol and mixtures thereof.

According to an embodiment, a composition lipoplexes of the invention may comprise a neutral lipid in an amount ranging from 0.5 mg to 14 mg in particular from 1 to 10 mg, and more particularly from 4 to 8 mg.

According to an embodiment, a composition lipoplexes of the invention may comprise from 0.05 to 1% and in particular from 0.1 to 0.5 % of neutral lipid by weight to the total weight of the total weight of the composition.

According to an embodiment, lipoplexes of the invention may be prepared in particular with DOTAP as cationic lipid and DOPE as neutral lipid.

According to an embodiment, the cationic lipid is not bis-(guadinium)-tren-cholesterol.

The nucleic acid sequences suitable for the invention may be of human, animal, vegetal, bacterial, viral or synthetic origin.

According to an embodiment, a nucleic acid sequence suitable for the invention may be chosen from a deoxyribonucleic acid sequence (DNA), a ribonucleic acid sequence (RNA), in particular a peptide nucleic acid sequence (PNA), in particular a small interfering RNA (siRNA) and mixtures thereof.

The deoxyribonucleic acid sequence may be genomic DNA or cDNA.

Ribonucleic acid sequence that may convene for the invention may be in particular chosen from mRNA, tRNA, rRNA, siRNA, short hairpin RNA (shRNA) or micro RNA (miRNA).

Peptide nucleic acids are artificially synthesized compounds chemically similar to DNA or RNA. They may be used in antisense therapy.

The nucleic acid sequences may be obtained by any technique known by the skilled person in the art and in particular by screening libraries, by chemical synthesis or by mixed method including chemical or enzymatic modifications of sequences obtained by screening libraries. A nucleic acid sequence suitable for the invention may be chemically modified.

With respect to the deoxyribonucleic or ribonucleic acid sequence, they may be single or double stranded.

The deoxyribonucleic acid sequences that may be useful for the instant invention may be of antisense type for decreasing the expression of a gene.

The deoxyribonucleic acid sequences may comprise therapeutic genes, regulatory sequences for transcription or for replication, modified or unmodified antisense sequences, regions for binding to other cellular components, etc. They may in particular direct the synthesis of a polypeptide specific for an infectious agent or may be capable of remedying a genetic or acquired deficiency.

For the purpose of the invention, the term "therapeutic gene" is intended to mean in particular any gene or any part of gene implied in the synthesis of a protein product having a therapeutic effect. The protein product thus encoded may be a protein, a peptide, etc. This protein product may be homologous with respect to the target cell (i.e. a product which is normally expressed in the target cell when said cell exhibits no pathology). In this case, the expression of a protein makes it possible, for example, to compensate for an insufficient expression in the cell or the expression of a protein that is inactive or weakly active due to a modification, or else to overexpress said protein. The therapeutic gene can also encode a mutant of a cellular protein, having increased stability, modified activity, etc. The protein product may also be heterologous with respect to the target cell. In this case, an expressed protein may, for example, add to or introduce a deficient activity in or into the cell, allowing it to combat a pathology or simulate an immune response.

Among the therapeutic products for the purpose of the present invention, mention may be made more particularly of enzymes, blood derivatives, hormones, lymphokines such as interleukins, interferons, TNF, etc., growth factors such as vascular endothelial growth factor, insulin-like growth factor and fibroblast growth factor, neurotransmitters or their precursors or enzymes for synthesizing them, trophic factors such as BDNF, CNTF, NGF, IGF, GMF, aFGF, bFGF, NT3, NT5 and HARP/pleiotrophin, dystrophin or a mini-dystrophin, utrophin, the cystic fibrosis-related CFTR protein, tumor suppressor genes such as p53, Rb, Rap1A, DCC and k-rev, genes encoding factors involved in clotting, such as factors VII, VIII and IX, genes involved in DNA repair, suicide genes (thymidine kinase, cytosine deaminase), genes for hemoglobin or other protein transporters, genes corresponding to the proteins involved in lipid metabolism, of apolipo-protein type chosen from apolipoproteins A-I, A-II, A-IV, B, C-I, C-II, C-III, D, E, F, G, H, J and Apo(A), metabolic enzymes such as, for example, lipoprotein lipase, hepatic lipase, lecithin cholesterol acyl-transferase, cholesterol 7-alpha-hydroxylase, phosphatidic acid phosphatase or lipid-transfer proteins such as cholesteryl ester transfer protein and phospholipid transfer protein, an HDL-binding protein or else a receptor chosen, for example, from LDL receptors, chylomicron-remnant receptors and scavenger receptors, erythropoietin, protein kinase C-3, etc.

The therapeutic nucleic acid may also be a siRNA, an antisense gene or sequence, the expression of which in the target cell makes it possible to control gene expression or cellular mRNA transcription. Such sequences may, for example, be transcribed, in the target cell, into RNAs that are complementary to cellular mRNAs, and thus block the translation thereof into protein. The therapeutic genes may also comprise the sequences encoding ribozymes, which are capable of selectively destroying target RNAs.

The nucleic acid sequences may also comprise one or more genes encoding an antigenic peptide capable of generating an immune response in humans or animals. In this particular embodiment, the invention therefore makes it possible to produce either vaccines or immuno-therapeutic treatments applied to humans or to animals, in particular against microorganisms, viruses or cancers. They may in particular be antigenic peptides specific for the Epstein Barr virus, for the HIV virus, for the hepatitis B virus, for the pseudorabies virus, for the syncitia forming virus or for other viruses, or else specific for tumors.

Preferably, the nucleic acid sequences may also comprises sequences which allow the expression of the therapeutic gene and/or of the gene encoding the antigenic peptide in the desired cell or organ. They may be the sequences which are naturally responsible for the expression of the gene under consideration when these sequences are capable of functioning in the infected cell. They may also be sequences of different origin (responsible for the expression of other proteins, or even synthetic sequences). In particular, they may be promoter sequences for eukaryotic or viral genes. For example, they may be promoter sequences derived from the genome of the cell that it is desired to infect. Similarly, they may be promoter sequences derived from the genome of a virus. In this regard, mention may be made, for example, of the promoters of the EIA, MLP, CMV and RSV genes, and tissue-specific promoters such as myosin chain promoters, for example, etc. in addition, these expression sequences can be modified by the addition of activation sequences, regulatory sequences, etc. They may also involve an inducible or repressible promoter.

Moreover, the nucleic acid may also comprise, in particular upstream of the therapeutic gene, a signal sequence which directs the synthesized therapeutic product into the target cell's secretion pathways. This signal sequence may be the natural signal sequence of the therapeutic product, but it may also be any other functional signal sequence, or an artificial signal sequence. The nucleic acid can also comprise a signal sequence which directs the synthesized therapeutic product to a particular compartment of the cell.

According to an embodiment, a nucleic acid sequence suitable for the invention may be a plasmid sequence.

Plasmid sequences suitable for the invention may be circular double-stranded DNA comprising a replication origin site for the production of the plasmid in bacteria strain, antibiotic resistance gene allowing the maintenance of the plasmid into bacteria under pressure of selection, an expression cassette comprising a therapeutic nucleic acid sequence under the control of a promoter and a transcription termination sequence.

As example of regulatory promoters of expression cassette, mention may be made of the early promoter of the simian virus SV40 or the early promoter of the cytomegalovirus (pCMV). Those regulatory promoters allow for constitutive expression of the transgene.

Alternatively regulatory promoters, whose expression may be induced or repressed with chemical compound, may be used. Tissue specific regulatory promoters may also be useful for the invention.

All those technologies are well known by the skilled person in the art.

The amount of nucleic acid sequence to be used in a nanocapsule of the invention may be adapted according to various factors, well known from the skilled person such as the cells or the tissues to be transfected *in vitro, in situ* or *in vivo,* the individual to be treated, the condition to be treated, the purpose of the therapy (gene or antisense therapy).

According to an embodiment, the ratio of amount of cationic and/or neutral lipids to nucleic acid sequence may range from 1:1:0.1 to 7:7:1, in particular from 2:2:0.3 to 4:4:0.6.

As for example, an amount of DNA may range from 100 µg to 2 mg, an amount of cationic lipid may range from 1 to 14 mg and an amount of neutral lipid may range from 1 to 14 mg.

### Nanocapsules

Nanocapsules that may be used in the invention may be obtained as described in EP 1 265 698.

The nanocapsules considered according to the invention comprise a lipid core that is liquid at room temperature, coated with an external lipid layer that is solid at room temperature.

Within the meaning of the invention, the expression "oily core" is to be intended to mean a continuous liquid fatty phase.

The oily core of the nanocapsules may be a fatty substance that is liquid at room temperature. For example, it may be chosen from triglycerides, fatty acid esters and mixtures thereof. It may exhibit a multilamellar organization.

The triglycerides that may be present in the oily core may be chosen from triglycerides comprising fatty acids ranging from C₈ to C₁₂.

Suitable triglycerides may be for example capric and caprylic acid triglycerides and mixtures thereof.

Fatty acid esters may be chosen from fatty acid esters ranging from C₈ to C_{18,} for example ethyl palmitate, ethyl oleate, ethyl myristate, isopropyl myristate, octyldodecyl myristate, and mixtures thereof. The fatty acid esters may in particular range from C₁₂ to C₁₆.

The oily core may represent from 20% to 60%, and in particular from 25% to 50% of the total weight of the nanocapsules.

The external lipid layer coating the nanocapsules may comprise at least one lipophilic surfactant.

The lipophilic surfactant may advantageously be solid at 20°C and liquid at about 37°C.

A lipophilic surfactant may be chosen from lecithin, polyglyceryl fatty acid(s) ester derivatives, and mixtures thereof.

Fatty acid(s) of the polyglyceryl fatty acid(s) esters derivatives may be chosen from C₈ to C₁₈ fatty acids, and in particular from C₁₂ to C₁₆ fatty acids.

According to an embodiment, a polyglyceryl-fatty acid ester derivative useful for the instant invention may be a polyglyceryl-6-dioleate.

According to an embodiment, a lecithin useful for the invention may be a lecithin whose proportion of phosphatidylcholine may be from 40% to 80%.

The amount of lipophilic surfactant contained in the external layer of the nanocapsules may be set such that the oily core to the lipophilic surfactant mass ratio may be chosen from 1 to 15, in particular from 1.5 to 13 and more particularly from 2 to 8.

The thickness of the external lipid layer may be from 2 to 10 nm.

The external lipid layer may comprise at least one non-ionic hydrophilic surfactant.

A non-ionic hydrophilic surfactant may be chosen from mixtures of free polyethylene glycol and polyglycol fatty acid(s) ester derivatives.

A non ionic hydrophilic surfactant suitable for the invention may be chosen from hydroxystearate derivatives and mixtures thereof.

In particular, a non-ionic hydrophilic surfactant may be a mixture of polyglycol mono- and di-esters of 12-hydroxystearic acid and free polyethylene glycol.

The non-ionic hydrophilic surfactant may represent from 2% to 10% by weight of the total weight of nanocapsules, in particular about 8%.

According to an embodiment, the oily fatty phase may be Labrafac^{®} WL 1349, the lipophilic surfactant may be Oleic Plurot^{®} or Lipoid^{®} S 75-3 and the non-ionic hydrophilic surfactant may be Solutol^{®} HS 15. These compounds have, the following characteristics:
- Labrafac^{®} WL 1349 (Gattefossé, Saint-Priest, France) is an oil composed of caprylic and capric acid (C₈ and C₁₀) medium-chain triglycerides. Its density is from 0.930 to 0.960 at 20°C. Its HLB value is about 1.
- Lipoid^{®} S 75-3 (Lipoid GmbH, Ludwigshafen, Germany) corresponds to soybean lecithin. Soybean lecithin contains about 69% phosphatidylcholine and 9% phosphatidylethanolamine.
- Oleic Plurol^{®} (GATTEFOSSE SA, St Priest, France) corresponds to polyglyceryl-6-dioleate.
- Solutol^{®} HS 15 (BASF, Ludwigshafen, Germany) is a polyethylene glycol-660 2-hydroxystearate.

According to an embodiment, a nanocapsule of the invention may be prepared, in particular, with caprylic and capric acid triglycerides as oily core, and a mixture of polyglyceryl-6-dioleate, free polyethyleneglycol and polyethyleneglycol-660-2-hydroxystearate for the external lipid layer.

Nanocapsules of the invention may have an average size in diameter of at least 40 nm in diameter, and in particular ranging from 50 to 200 nm, in particular ranging from 55 to 155 nm and more particularly ranging from 90 to 110 nm in diameter.

As stated previously, the nanocapsules according to the invention may exhibit a zeta potential near 5 ± 0.4 mV.

It has to be noticed that the zeta potential is distinct from the surface charge. The potential zeta is depending on factors which correspond to a ratio between the thickness of the double electric layer, of the diameter of the particle, and of the form of the particle.

The zeta potential may be determined by well-known techniques in the art.

In the instant invention, the potential zeta measure may be carried out by laser Doppler interferometry (zeta sizer 3000 HSA) according to manufacturer recommandations.

The nanocapsule of the invention sequence may present a zeta potential ranging from -10 to 15 mV, in particular ranging from -5 to 10 mV, and more particularly ranging from 0 to 7 mV.

According to another embodiment, the nanocapsules of the invention may present a potential zeta of about 5 +/- 0.4 mv.

According to another embodiment, the nanocapsules of the invention may present an isoelectric point pl ranging from about 6.5 to about 7.8 and in particular being about 7.5+/-0.13.

The isoelectric point of nanocapsules of the invention may be measured according to any suitable electrophoretic mobility measurement methods known in the art.

### Process of preparation of the nanocapsules

A process for preparing nanocapsules in accordance with the invention may be a process as described in EP 1 265 698.

A process of the invention may comprise at least the following steps of:
a) preparing an oil/water emulsion comprising an oily fatty phase, a lipophilic surfactant solid at 20°C, a non-ionic hydrophilic surfactant an aqueous dispersion of lipophilic complexes of at least one nucleic acid sequence, said complexes having a positive/negative charge ratio of at least 0.5,
b) subjecting the emulsion of step a) to a cycle of temperature comprising an increase of temperature up to a temperature T₂ greater than a phase inversion temperature for obtaining a water/oil emulsion, followed with a decrease of temperature down to T₁, lower than the phase inversion temperature, said cycle of temperature inducing a phase inversion of said emulsion,
c) reiterating at least one cycle of temperature according to step b),
d) subjecting the emulsion obtained at step c) to a fast cooling-dilution step with water at 0 °C for obtaining a nanocapsule in water.

The number of cycles to be applied to the emulsion depends on the amount of energy required to form the nanocapsules. A number of cycles may range, for example, from 2 to 10, or from 3 to 8, or may be of 5.

The phase inversion between the oil/water emulsion and the water/oil emulsion is reflected by a reduction in the conductivity when the temperature increases until it is canceled out. The average temperature of the phase inversion zone corresponds to the phase inversion temperature (PIT). The organization of the system in the form of nanocapsules is reflected visually by a change in the appearance of the initial system, which changes from opaque-white to translucent-white. This change takes place at a temperature below the PIT. This temperature is generally between 6 and 15°C below the PIT.

T1 is a temperature at which the conductivity is at least equal to 90-95% of the conductivity measured at 20°C.

T2 is the temperature at which the conductivity becomes canceled out.

The step d) comprising a sudden cooling (or quench) of the oil/water emulsion to a temperature in the region of T1, preferably above T1 may be conducted under magnetic stirring, by diluting the emulsion from threefold to tenfold using deionized water at 2°C ± 1°C added to the fine emulsion. The particles obtained are kept stirring for 5 minutes.

In one embodiment, the oily phase may be a triglyceride, the solid lipophilic surfactant may be polyglyceryl-6-dioleate and the non-ionic hydrophilic surfactant may be a mixture of free polyethyleneglycol and of polyglycol mono- and di-esters of 12-hydroxystearic acid.

The oil/water emulsion advantageously may comprise from 1% to 5% of lipophilic surfactant, from 5% to 15% of non-ionic hydrophilic surfactant, from 5% to 15% of oily fatty phase, from 64% to 89% of an aqueous dispersion comprising from 1 to 15 % of lipophilic complexes of at least one nucleic acid sequence. The percentages are expressed on a weight basis. The relative amount of each component is adjusted so as to the total amount reaches 100%.

According to an embodiment, the aqueous dispersion of lipophilic complexes may be an aqueous dispersion of lipoplexes. The lipoplexes may be as above-defined.

Lipoplexes may be obtained by any known technique of the art.

For example, cationic and optionally neutral lipids may be formulated as liposomes according to any known techniques, or under micellar or threadlike micelles or ribbons and the obtained supramolecular assembly may be mixed with the nucleic acid sequence to be complexed.

According to an embodiment, the aqueous phase of the oil/water emulsion may also contain 1% to 4% of a salt, for instance sodium chloride. Changing the salt concentration may bring about a shift in the phase inversion zone. The higher the salt concentration is, the lower the phase inversion temperature may be.

### Pharmaceutical compositions and method of treatment

The nanocapsules according to the invention may also comprise one or more targeting elements for being more accurately and efficiently directed to receptors or ligands at the surface of the cell.

By way of example, the nanocapsules of the present invention may comprise one or more antibodies directed against cell surface molecules, or else one or more membrane-receptor ligands such as insulin, transferrin, folic acid or any other growth factor, cytokines or vitamins. The nanocapsules may use modified or unmodified lectins in order to target specific polysaccharides at the surface of the cell or on the neighboring extracellular matrix. Proteins containing a RGD unit, peptides containing a tandem of RGD units, which may or may not be cyclic, and also polylysine peptides or ligand peptides, which may be natural or synthetic, can thus be used.

The pharmaceutical compositions of the invention may comprise a vehicle that is pharmaceutically acceptable, in particular for an injectable formulation, in particular for injection directly into the desired organ or for topical administration, for example to the skin and/or mucous membranes. They may be sterile isotonic solutions or dry, in particular lyophilized, compositions which, by means of the addition, according to the case, of sterilized water or of physiological saline, make it possible to constitute injectable solutes.

It is clear that the doses of nucleic acid used for the injection and also the number of administrations may be adjusted by means of various parameters, and in particular as a function of the method of administration under consideration, of the pathology involved, of the nature of the gene to be expressed or repressed, or of the nature of the siRNA used to inhibit a gene expression, of the individual to be treated, and of the conditions to be treated or prevented.

Within the meaning of the invention, the term "to prevent" with respect to a disease is to be understood as meaning to reduce the risk of occurrence of said disease.

As regards more particularly the method of administration, it may involve either direct injection into the tissues or the circulatory system, or treatment of cells in culture followed by reimplantation in vivo by injection or graft.

The claimed composition proves to be particularly advantageous for internal administration.

For the purpose of the present invention, the term "internal administration" means that the claimed compositions are compatible with administration into the tissue of an organism, for example a muscle, intra-dermally or subcutaneously. Furthermore, topical, oral, pulmonary, nasal and mucosal, such as, for example, buccal, vaginal or rectal, administration may be used.

The compositions according to the invention are particularly advantageous from a therapeutic point of view.

Thus, the potential applications are in the field of gene or antisense or gene silencing therapy.

Another application comes from the field of immunization. In this case, a DNA encoding a bacterial, viral or other antigen is injected into cells, preferably muscle cells or dendritic cells. Insofar as the claimed compositions are particularly advantageous for increasing the amount of proteins synthesized by the transfected cells, it is possible, by virtue of this, to obtain higher concentrations of antibodies and of cytotoxic T lymphocytes.

The invention also relates to a method for the cellular transfection *in vivo* of at least one nucleic acid sequence, characterized in that said nucleic acid sequence is administered within a nanocapsule as above-defined to an individual in need thereof.

The administration can be carried out topically, directly into the cells under consideration, or by means of one of the routes of administration discussed above.

The present invention will be described more fully by means of the examples and figures which follow, which should be considered as non-limiting illustrations.

### Legends of figures

Figure 1: a-d) Gallery of nanocapsules containing DNA molecules (black arrows). Scale bar is 50 nm
Figure 2: Complement consumption at 37°C by empty nanocapsules, DOTAP-DOPE/DNA lipoplexes and DNA nanocapsules. The standard deviation results from three different experiments.
Figure 3: Visualization of endocytosis by confocal microscopy in presence of serum, (a): HeLa cells, (b): HeLa cells incubated with DOTAP-DOPE/DNA lipoplexes, (c) and (d): HeLa cells incubated with DNA nanocapsules (a, b and c images result from Nomarsky contrast, d image results from the superposition of Nomarsky images and fluorescent acquisitions).
Figure 4: Evolution of fluorescent empty and DNA/nanocapsules in blood during 1 h. Data are expressed in percentage of injected dose and are the mean of three rats ± sem.
Figure 5: Expression of luciferase after gene transfer into COS7 with DOTAP/DOPE-DNA lipoplex or DNA/nanocapsules. Nanocapsules 1 comprise as lipophilic surfactant Oleic Plurol^{®}, and nanocapsules 2 comprise as lipophilic surfactant Lipoid^{®} S75-3.

### EXAMPLES

### Example 1

### Preparation of nanocapsules with or without DNA complex

For the preparation of complexes of DNA with cationic and neutral lipids, DOTAP (1,2-Dioleoyl-3-Trimethylammonium-Propane) and DOPE (L-α-Dioleoyl Phosphatidy1ethanolarnine) (Avanti Polar Lipids, Inc, Alabaster, USA) were first dissolved in chloroform (Sigma Aldrich Chemie Gmbh, Steinheim, Germany) and then dried by evaporation process under vacuum. The lipid film was hydrated with deionized water. Then liposomes were sonicated for 10 minutes. Lipoplexes were prepared by mixing DOTAP-DOPE (1-1, M/M) liposomes with 660 µg of plasmid encoding luciferase (Ferrari et al., Gene Ther., 1997, 4: 1100-1106.) at a charge ratio of 5 (+/-) in 150 mM NaCl.

The formulation of nanocapsules was based on a phase inversion process described by Heurtault et al. (Pharm Res 2002;19(6):875-80). Nanocapsules were composed of lipophilic Labrafac^{®} WL 1349 (caprylic-capric acid triglycerides, European Pharmacopia, IVth, 2002), Oleic Plurol^{®} (Polyglyceryl-6 dioleate) and Solutol^{®} HS-15 (70% of PEG 660 hydroxystearate (HS-PEG) and 30% of free PEG 660 Da, European Pharmacopia, IVth, 2002) which was gift from BASF (Ludwigshafen, Germany).

Briefly, 3.9 % of Oleic Plurol^{®} (w/w), 5.9 % of Solutol^{®}(w/w), 9.9 % of Labrafac^{®} (w/w), 78.9 % of water (w/w) and 1.4 % of NaCl, were mixed together under magnetic stirring.

For the preparation of DNA/nanocapsules, the lipoplexes were added to the water of the emulsion for obtaining an aqueous dispersion comprising an amount of lipoplexes of 2.75% before performance of the formulation process.

Three temperature cycles were applied to reach the phase inversion, between 40° and 60°C, from an oil-in-water to a water-in-oil emulsion. Thereafter, the mixture underwent a fast cooling-dilution process with water at 0°C, leading to the formation of nanocapsules in water.

Fluorescent lipid nanocapsules were obtained by using fluorescent Labrafac^{®} obtained by adding 500 µl Labrafac^{®} WL 1349 with 500 µl of a solution containing 2 µg/µl of Nile Red (Sigma Aldrich Chemie Gmbh, Steinheim, Germany) in acetone. Before use, acetone was evaporated.

### Example 2

### Electrophoretic and Dynamic light scattering

The average volume diameter and the polydispersity index of the nanoparticles were determined by dynamic light scattering using a Malvern Autosizer 4700 (Malvern Instruments S.A., Orsay, France) fitted with a 488nm laser beam at a fixed angle (90°) at 25°C.

A 1:400 dilution of the nanoparticle suspensions in distilled water was achieved in order to enable measurements (performed in triplicate). Particles were dispersed in deionized water and analyzed at 25°C. The mean diameter of empty nanocapsules was of 51 +/- 8 nm, and the mean diameter of Lipoplexes was of 400 nm +/-100 nm, whereas the mean diameter of DNA/nanocapsules was 110 ± 55 nm.

The zeta potential and the electrophoretic mobility measurements of the nanoparticles were performed using a Malvern Zetasizer 3 (Malvern Instruments, Malvern, England) equipped with an AZ-4 cell and were based on the laser doppler effect. The measure of zeta potential was achieved on nanoparticle suspensions diluted to ImM of NaCl and at pH 7.4. The analysis was made at a voltage of 150V.

Electrophoretic mobility measurements were realized as a function of NaCl concentration for pHs between 3 to 10, by using MPT 1 titrator (Malvern Instruments, Malvern, England). To set the pH at the desired value, the electrolytes used were sodium hydroxide (0.1 M) and hydrochloride acid (0.1 M). Measurements were made in water at 25°C, with dielectric constant of 79, refractive index of 1.33, viscosity of 0.89 cP, cell voltage 150 V and current of 5 mA. Nanocapsules suspension was diluted in deionized water and five NaCl concentrations were used: 1, 10, 25, 50, 100 and 150 mM. Considering that NaCl was used in the formulation of the nanocapsules, NaCl concentrations in the final suspension were adjusted using a conductimeter Cond. 330i / SET (VTW, Weilheim, Germany). Each sample was diluted in deionized water at 1:150 (v/v) and conductivity was measured at room temperature and under magnetic stirring.

The DNA/nanocapsules had a zeta potential of 5 ± 0.4 mV and a *pI* of 7.50 +/-0.13 compared to empty nanocapsules having a zeta potential of-37 ± 0.3 mV and a *pI* of 3.40 ± 0.13 and DOTAP-DOPE-DNA lipoplex having a zeta potential of 50 ± 0.8 and a *pI* 9.5 ± 0.13.

### Example 3

### Cryo-transmission electron microscopy (cryo-TEM)

DNA-nanocapsules were dialyzed in a dialysis bag Spectra/Por^{®} membrane 50,000 Da (Spectrum Laboratories, Inc, USA), *vs* deionized water (400ml) at room temperature and under magnetic stirring during 96 hours. Water was not changed during the experiment. Five µl samples were deposited onto a holey carbon coated copper grid; the excess was blotted with a filter paper, and the grid was plunged into a liquid ethane bath cooled with liquid nitrogen (Leica EM CPC). Specimens were maintained at a temperature of approximately 170°C, using a cryo holder (Gatan), and were observed with a FEI Tecnai F20 electron microscope operating at 200 kV and at a nominal magnification of 50,000X under low-dose conditions. Images were recorded with a 2K x2K Gatan slow scan CCD camera.

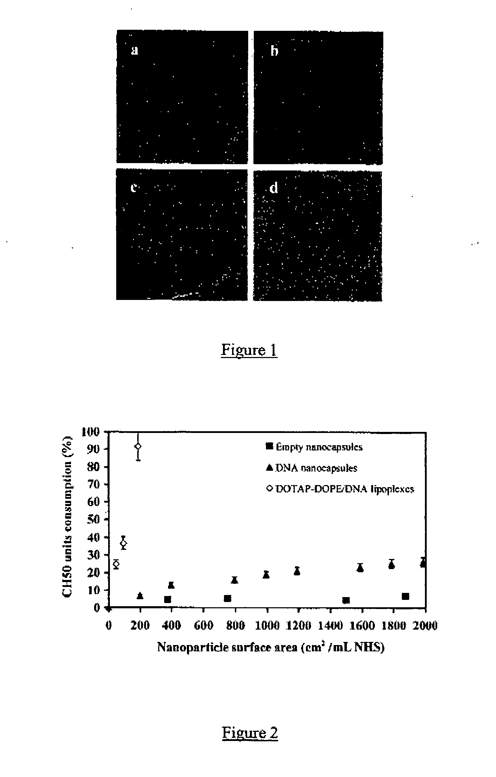

Cryo-EM observations of the formulation of nanocapsules synthesized in the presence of DOTAP/DNA lipoplexes revealed large core-shell nanocapsules (diameter ranging from 50 to 200 nm, Figures 1 a-d). The dense core is made of multilamellar organization exposing a repetitive spacing of about 5.6 nm that likely corresponds to DOTAP-DOPE/DNA lipoplexes. The latter is surrounded by a homogeneous density shell suggesting that the lipoplexe core is embedded within a neutral lipid shell. The DOTAP-DOPE/DNA lipoplexes core had a various size ranging from 30 nm to 150 nm.

### Example 4

### Measure of the complement activation: CH50 technique

Complement consumption was tested on normal human serum (Etablissement Français du Sang, Angers, France) by measuring the residual hemolytic capacity of the complement system after contact with the different particles. (Kazatchkine et al., Techniques du complement. Paris: Inserm,22-23; 1986; and Mayer, Experimental Immunochemistry, Springfield, IL, USA: Thomas; 1961. 133-156 p.)

The technique consisted in determining the amount of serum able to lyse 50% of a fixed number of sensitized sheep erythrocytes with rabbit anti-sheep erythrocyte antibodies (CH50), according to the following procedure.

Complement consumption was tested in normal human serum (NHS) by measuring the residual hemolytic capacity of the complement after contact with the different particles. The technique consists of the determination of the amount of serum able to lyse 50% of a fixed number of sensitized sheep erythrocytes (CH50) (Mayer MM. Experimental Immunochemistry, Kabat EAaM, M.M, 1961. p. 133-156.). VBS (Veronal buffered saline) and VBS containing 0.15mM Ca²⁺ and 0.5mM Mg²⁺ (VBS²⁺) were prepared as described elsewhere (Kazatchkine et al. Techniques du complement, 1986). NHS was a pool of forty donor blood samples provided by the "Etablissement Français du Sang" (Angers, France), aliquoted and stored at -80 °C until use. In order to activate the complement via the classical pathway, sheep erythrocytes (Sheep erythrocytes 50%, Biomérieux, Marcy-l'Etoile, France) were sensitized by rabbit anti-sheep erythrocyte antibodies (Hemolytic serum, Biomérieux, Marcy-l'Etoile, France) at a final dilution of 1/800 (v/v) in VBS²⁺. Antibody-activated sheep erythrocytes were prepared at 1.10⁸ cells/mL in VBS²⁺. To assess consumption of CH50 units in the presence of particles during a constant incubation time, increasing amounts of particle suspensions were added to NHS diluted in VBS²⁺ so that the final dilution of NHS in the reaction mixture was 1/4 (v/v) in a final volume of ImL. Suspensions were incubated at 37°C with gentle agitation to allow the reaction between serum components and DNA/nanocapsules. Then, these suspensions were diluted with VBS²⁺ before mixing with a suspension of sensitized sheep erythrocytes. After an incubation at 37°C under gentle stirring, the reaction was stopped by addition of ice cold NaCl (0.15 M). Unlysed erythrocytes formed a deposit after 10 min at 500 g. The optical density (OD) of 200 µL supernatant, related to the lytic capacity of the serum, was recorded at 415 nm using a microplate reader (Multiskan Anscent, Labsystems SA, Cergy-Pontoise, France) and compared to the results obtained with control serum.

The complement activation was expressed in terms of CH50 units consumption and was plotted as a function of the surface area in order to compare particles with different mean diameters. A test using a solution of 1.5 mM-ethylene glycol bis(2-aminoethyl ether)-N,N,N'N'-tetraacetic acid/Mg²⁺ (EGTA/Mg²⁺) (Liu D et al., Pharm Res 1995;12(11):1775-1780) which acted as an inhibitor of the classical pathway by chelating Ca²⁺ ions, was carried out.

All experiments were performed in triplicate. A t-test analysis of two non-matched samples was used to test for the statistical significance of the results.

The activation of this system was assessed *in vitro* at a fixed amount of human serum in the presence of an increasing surface area of particles (Fig. 2).

As shown in Figure 2, the consumption of CH50 units by complexes of DOTAP-DOPE/DNA was linear and dose-dependent, and reached 100% at approximately 200 cm², despite the presence of DOPE, which would decrease this activation. (Plank, Hum Gene Ther 1996;7(12):1437-46). This strong activation was attributed to cationic liposomes.

Encapsulating lipoplexes into nanocapsules, prevent opsonization by proteins of the complement system, and lead to a dramatically drop in the complement activation, 7% of CH50 units consumption at 200 cm². The activation did not exceed 26% at 2000 cm² (Figure 2).

Empty nanocapsules induced lower activation than that of DNA/nanocapsules, which may be due to their lower size.

### Example 5

### Nanocapsules internalization

HeLa cells were cultured in Dulbecco's Modified Eagle Medium (DMEM) medium containing 10% FCS (Cambrex Biosciences, Verviers, Belgium) and 1% antibiotics (Cambrex Biosciences, Verviers, Belgium) in cell chamber (Lab-Tek^{™} Chamber Slide^{™} System, Nunc, Wiesbaden, Germany) at a concentration leading to a confluence at day 4. At day 3, the medium was totally removed and fluorescent DNA/nanocapsules were added to 500µl of medium in each chamber. After two-hours incubation, 500µl of medium was added. At day 4, the medium was removed and cells were washed four times with phosphate buffer saline (PBS), then fixed with paraformaldehyde (PFA) 4% (Sigma Aldrich Chemie Gmbh, Steinheim, Germany) during 15 minutes at 4°C and washed three times with PBS. Cells and fluorescent nanocapsules were observed at room temperature by confocal microscopy (Olympus light microscope Fluoview FU 300 Laser Scanning Confocal Imaging System (Paris, France) with a helium argon laser (λₑₓ : 543 nm, λ_{cm}: 572 nm). The morphology of HeLa cells was obtained by Nomarsky contrast.

Nanocapsules labeled with a fluorescent marker were observed by confocal microscopy 24 hours after incubation with HeLa cells.

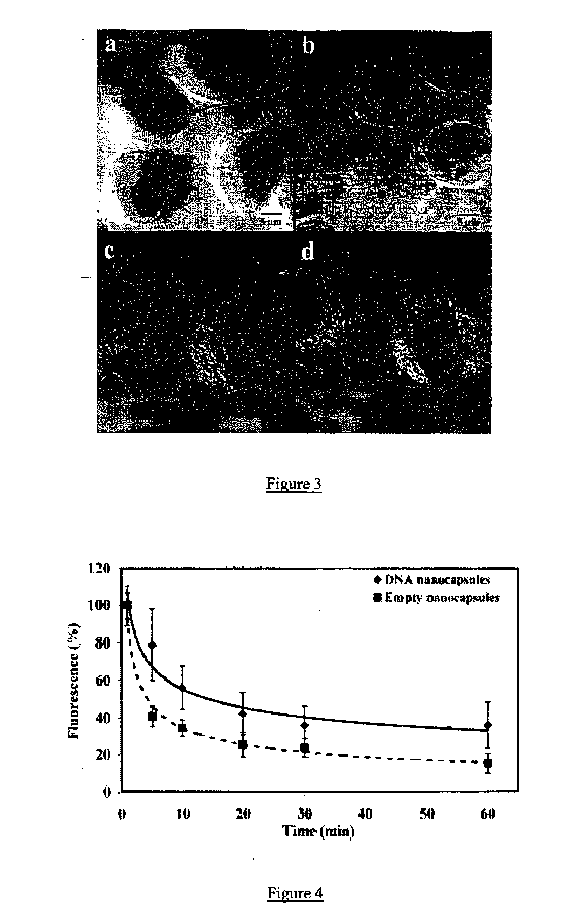

The internalization of DNA/nanocapsules seemed not to be hindered by the serum, neither by the PEG although it may decrease it (Figure 3 c and d).

### Example 6

### Determination of the blood half-life

Animal care was administrated in strict accordance to French Ministry of Agriculture regulation.

One hundred and fifty microliters of fluorescent nanocapsules were injected in the caudal vein of female Swiss mice (20-22g) (n=18) (Ets Janvier, Le Genest-St-ile, France). The fluorescence was measured at time 0, 1, 5, 10, 20, 30 and 60 minutes. For each time, a blood sampling (0.5 to 1 ml) was performed by cardiac puncture on three mice and each sample was centrifuged in a venous blood collection tube (Vacutainer, SST II Advance, 5 ml, Becton Dickinson France SAS, Le Pont-De-Claix, France), at 2000g and at room temperature during 10 minutes.

Animals were sacrificed at the end of the experiments. One hundred and fifty microliters of the supernatant were deposited in a black 96-well plate (Greiner Bio-one, Frickenhausen, Germany). Blank samples were constituted by the supernatant of centrifuged blood taken on three not-injected mice. It represented the residual fluorescence of the plasma. The fluorescence was counted (ex: 450nm, em: 595nm) by a fluorimeter (Fluostar, BMG Labtechnologies, Champigny sur Marne, France) and results were analyzed with the software BIOLISE 2.0. The fluorescence was expressed in fluorescent units (FU) and was calculated as: FUₛₐₘₚₗₑ - FU_{blank}. The 100% of fluorescence was considered as the value at t=1 min.

Figure 4 showed that empty nanocapsules were more rapidly removed from blood that DNA/nanocapsules, t_{1/2} = 5 and 15 minutes, respectively. Moreover, at one hour, approximately 35% of DNA/nanocapsules were still in blood circulation in comparison to 20% of empty nanocapsules and to 1% in blood at 5 min for free lipoplexes.

### Exemple 7

### Cells transfection with DNA/nanocapsules

Lipoplexes, nanocapsules 1 (Oleic plurol^{®}) and nanocapsules 2 (Lipoid^{®} S75-3) were prepared as described in example 1.

The COS7 cells were cultured in flasks, at 37 °C in 5% CO₂/humidificated atmosphere, in D-MEM with Glucose, 1-Glutamine and Pyruvate supplemented with 1% streptomycin/penicillin (GIBCO, Invitrogen Life technologies, Carlsbad, CA, USA) and 10% fetal calf serum FCS (Eurobio, Courtaboeuf, France). At day -1 before transfection, the cells were transferred onto 24-well culture plates, at 65000 cells/well, resulting in approximately 70-80 % confluence 24 hours later. Transfection was performed by adding to each well, 50 µL of complexes or nanocapsules in 500 µL of serum-free D-MEM. After 2h, the transfection medium was replaced by 1 mL of D-MEM containing 10% FCS and 1% streptomycin/penicillin. Transfection experiments were performed in triplicate and luciferase expression was evaluated 24 hours after transfection. Luciferase activity was measured with Victor² (PerkinElmer), using a Luciferase Assay System (Promega) Luciferase activity was determined by measuring the light emission after addition of 100 µl of luciferase assay substrate to 10 µl of supernatant.

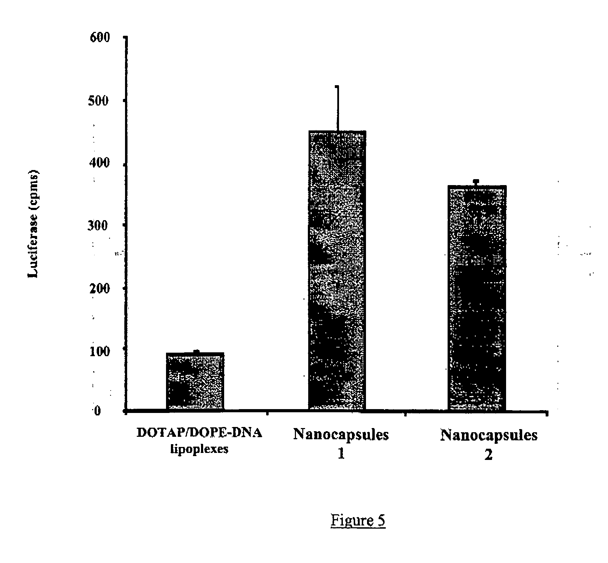

The luciferase activity was highly increased with nanocapsules 1 or 2 in comparison with lipoplexes (Figure 5).

## Claims

1. A nanocapsule comprising at least:
- an external lipid layer, solid at room temperature,
- an oily core, liquid room temperature,
said oily core containing at least one lipophilic complex of at least one nucleic acid sequence, said complex having a positive/negative charges ratio of at least 0.5.

2. The nanocapsule according to claim 1 wherein said complex presents a zeta potential ranging from 40 to 60 mV, in particular ranging from 45 to 55 m V, and more particularly ranging from 48 to 52 mV.

3. The nanocapsule according to anyone of the preceding claims, wherein said complex has a positive/negative charges ratio ranging from 0.5:1 to 10:1, in particular from 1:1 to 5:1, and more particularly being of 5:1.

4. The nanocapsule according to anyone of the preceding claims, wherein said complex is present in an amount of up to 50%, in particular from 7 to 35%, and more particularly from 10 to 25% by weight relative to the total weight of the nanocapsule.

5. The nanocapsule according to anyone of the preceding claims, wherein said complex is a lipoplex comprising at least one cationic lipid and optionally at least one neutral lipid.

6. The nanocapsule according to the preceding claim wherein the cationic lipid contains at least one ammonium in moiety.

7. The nanocapsule according to claim 4 or 5, wherein the cationic lipid is chosen from DOTAP, DOTMA, DMRIE, DOSPA, DOGS, DODAP, DODAB, CTAB, 1-DPPES, aminoglycoside lipidic derivatives , and mixtures thereof.

8. The nanocapsule according to anyone of claims 5 to 7, wherein the neutral lipid is chosen from DOPE, DOPC, cholesterol, and mixtures thereof.

9. The nanocapsule according to anyone of the preceding claims, wherein the nucleic acid sequence is chosen from a deoxyribonucleic acid sequence (DNA), a ribonucleic acid sequence (RNA), a peptide nucleic acid sequence (PNA) and mixtures thereof.

10. The nanocapsule according to anyone of the preceding claims, wherein the external lipid layer comprises at least one lipophilic surfactant.

11. The nanocapsule according to the preceding claim, wherein the lipophilic surfactant is chosen from lecithin, polyglyceryl fatty acid(s) ester derivatives and mixtures thereof.

12. The nanocapsule according to the preceding claim, wherein the fatty acid(s) is/are chosen from C₈ to C₁₈ fatty acids, and in particular from C₁₂ to C₁₆ fatty acids.

13. The nanocapsule according to claim 11 or 12 wherein the ester polyglyceryl fatty derivative is a polyglyceryl-6-dioleate.

14. The nanocapsule according to anyone of the preceding claims, wherein the external lipid layer comprises at least one non-ionic hydrophilic surfactant.

15. The nanocapsule according to the preceding claim, wherein the non ionic hydrophilic surfactant is chosen from mixtures of free polyethylene glycol and polyglycol fatty acid(s) ester derivatives.

16. The nanocapsule according to claim 14 or 15, wherein the non ionic hydrophilic surfactant is chosen from hydroxystearate derivatives and mixtures thereof, and in particular is a mixture of polyglycol mono- and di-esters of 12-hydroxystearic acid and free polyethylene glycol.

17. The nanocapsule according to anyone of the preceding claims, wherein the oily core is chosen from triglycerides, fatty acid esters, and mixtures thereof.

18. The nanocapsule according to the preceding claim, wherein the triglycerides comprises fatty acids ranging from C₈ to C₁₂.

19. The nanocapsule according to claim 17, wherein fatty acid esters are chosen from fatty acid esters ranging from C₈ to C₁₈.

20. The nanocapsule according to anyone of the preceding claims, wherein the oily core is representing from 20 to 60 %, in particular from 25 to 50 % of the total weight of the nanocapsule.

21. The nanocapsule according to anyone of the claims 10 to 20, wherein the mass ratio of oily core to the lipophilic surfactant is from 1 to 15, in particular from 1.5 to 13, and more particularly from 2 to 8.

22. The nanocapsule according to anyone of the preceding claims, wherein the nanocapsule has an average size of at least 40 nm in diameter, and in particular ranging from 50 to 200 nm, in particular ranging from 55 to 155 nm, and more particularly ranging from 90 to 110 nm in diameter.

23. The nanocapsule according to anyone of the preceding claims, wherein said nanocapsule presents a zeta potential ranging from -10 to 15 mV, in particular ranging from -5 to 10 mV and more particularly ranging from 0 to 7 mV.

24. The nanocapsule according to anyone of the preceding claims, wherein said nanocapsule has an isoelectric point ranging from 6.5 to 7.5.

25. A use of a nanocapsule comprising (a) an external layer solid at room temperature and (b) an oily core, liquid at room temperature, for encapsulating at least one lipophilic complex of at least one nucleic acid sequence, said complex having a positive/negative charges ratio of at least 0.5.

26. A method for introducing *in vitro* and/or *ex vivo* at least one nucleic acid sequence into a host cell comprising at least a step of contacting a nanocapsule according to anyone of claims 1 to 23 with a host cell in conditions suitable for the entry of a nucleic acid sequence comprised into said nanocapsule into said host cell.

27. A pharmaceutical composition comprising in a pharmaceutically acceptable medium at least one nanocapsule according to anyone of claims 1 to 24.

28. A use of a nanocapsule according to anyone of claims 1 to 24 for the manufacture of a pharmaceutical composition intended to be used in gene or antisense or gene silencing therapy.

29. A process for obtaining a nanocapsule according to anyone of claims 1 to 24 comprising at least the steps of:
a) preparing an oil/water emulsion comprising an oily fatty phase, a lipophilic surfactant solid at 20°C, a non-ionic hydrophilic surfactant, an aqueous dispersion of lipophilic complexes of at least one nucleic acid sequence, said complexes having a positive/negative charge ratio of at least 0.5,
b) subjecting the emulsion of step a) to a cycle of temperature comprising an increase of temperature up to a temperature T₂ greater than a phase inversion temperature for obtaining a water/oil emulsion, followed with a decrease of temperature down to T₁, lower than the phase inversion temperature, said cycle of temperature inducing a phase inversion of said emulsion,
c) reiterating at least one cycle of temperature according to step b),
d) subjecting the emulsion obtained at step c) to a fast cooling-dilution step with water at 0 °C for obtaining a nanocapsule in water.

30. The process according to the preceding claim, wherein the oil/water emulsion comprises:
- 1 to 5% of lipophilic surfactant.
- 5 to 15% of non-ionic hydrophilic surfactant,
- 5 to 15% of liquid fatty phase,
- 64 to 89% of an aqueous dispersion comprising from 1 to 15% of lipophilic complexes, the percentage being expressed on a weight basis.

31. The process according to anyone of claims 29 or 30, wherein the complexes of said aqueous dispersion of complexes are lipoplexes, as defined according to anyone of claims 5 to 9.
